# EUROPÄISCHE PATENTANMELDUNG

(11) **EP 0 887 409 A1**
(43) Veröffentlichungstag der Anmeldung: **30.12.1998**
(21) Anmeldenummer: 97110464.1
(22) Anmeldetag: 26.06.1997
(51) Int. Cl.: C12N 15/19, C07K 14/52, G01N 33/68, C12N 1/21, A61K 38/19, C12Q 1/68, A61K 31/70

(54) **Chemokin MIG-beta: cDNA Fragment, Protein, Expressionsvektor, transformierte Zelle, Diagnoseverfahren und Arzneimittel**

(71) Anmelder: Werner, Ernst, 6020 Innsbruck (AT); Werner, Gabriele, 6020 Innsbruck (AT)
(72) Erfinder: Werner, Ernst, 6020 Innsbruck (AT); Werner, Gabriele, 6020 Innsbruck (AT)
(74) Vertreter: Torggler, Paul Norbert

(57) **Zusammenfassung**

Die Erfindung betrifft ein cDNA-Fragment wie es im Sequenzprotokoll mit SEQ ID NO:1 bzw. in bestimmten Teilen davon definiert ist. Außerdem betrifft die Erfindung ein entsprechendes Protein sowie dessen Verwendung in Diagnose und Therapie.

## Beschreibung

Die Erfindung betrifft ein cDNA-Fragment, ein Protein, einen DNA-Transfervektor, eine Zelle, ein Diagnoseverfahren, ein Arzneimittel sowie die Herstellung von Arzneimitteln.

Chemokine sind kleine Zytokine (ca. 10 kDa groß), die die Wanderung von Leukozyten aktivieren und lenken können. Besondere Aufmerksamkeit erhielten Chemokine im letzten Jahr vor allem dadurch, daß ihre Rolle in der HIV-1 Infektion entdeckt wurde. So kann z. B. das Chemokin SDF-1, das der CxC-Klasse angehört, die HIV-1 Infektion blockieren (Referenzen 1 und 2).

Im Rahmen der Klonierung eines durch Interferon-gamma induzierten Proteins in humanen Myelomonocytomazellen (THP-1) wurde eine neue, durch Interferon-gamma in diesen Zellen induzierte mRNA mit der Größe von 1445 Bp isoliert. Diese mRNA enthält einen Leserahmen für ein 94 Aminosäuren langen, bislang nicht bekanntes Protein (10.4 kDa). Der Leserahmen erstreckt sich von der Lage 46 bis zur Lage 327 in der im Sequenzprotokoll SEQ ID NO:1 angegebenen Nukleotid-Sequenz, welches Sequenzprotokoll integrierender Bestandteil der folgenden Beschreibung ist.

Das Protein selbst ist im Sequenzprotokoll SEQ ID NO:2 wiedergegeben.

Ein Vergleich mit bekannten Proteinsequenzen ergibt als stärkste Homologie eine Homologie mit einem als Mig bzw. IP-10 bezeichneten Chemokin, gefolgt von anderen Chemokinen der CxC-Klasse, weshalb für das der vorliegenden Anmeldung zugrunde liegende Protein die Bezeichnung 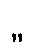 Mig-β" bzw 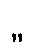 Mig-beta" gewählt wurde. Ein Übersichtsartikel zu den genannten Chemokinen Mig bzw. IP-10 ist die Referenz 3.

In Übereinstimmung damit ergibt eine Suche nach Proteinstrukturmotiven in der Prosite Database das Chemokin CxC-Motiv als einzige signifikantes Motiv. Aus dieser eindeutigen Homologie und aus der Induktion durch Interferon-gamma kann klar geschlossen werden, daß das neue Protein Mig-β der Chemokin-Klasse angehört und einen wichtigen Mediator der Immunantwort darstellt.

Beim erfindungsgemäßen Protein gemäß SEQ ID NO:2 erstreckt sich die Signalsequenz bzw. das Signal-Peptid mit hoher Wahrscheinlichkeit über die ersten 21 Aminosäuren. Die Erfindung umfaßt sowohl das gesamte Protein wie auch das Protein ohne das Signal-Peptid.

Die Erfindung umfaßt auch DNA-Transfervektoren, die eine cDNA-Sequenz enthalten, die einem Gen oder DNA-Fragment entsprechen, wie es in den Ansprüchen 1 bis 3 definiert ist. Ein solcher DNA-Transfervektor kann beispielsweise ein bakterielles Plasmid oder eine Bakteriophagen DNA sein. Vom Schutzumfang umfaßt ist auch eine Zelle, die von einem solchen Transfervektortransformiert ist.

Darüber hinaus umfaßt die Erfindung DNA-Fragmente bzw. DNA-Transfervektoren, bei denen im Sequenzprotokoll angegebene Codons durch andere Codons ersetzt sind, welche aber für die selben Aminosäuren codieren.

Für die erfindungsgemäße cDNA bzw. die entsprechende mRNA oder das entsprechende Protein gibt es zahlreiche Anwendungsmöglichkeiten. Eine davon besteht in einem Diagnoseverfahren, bei dem in einer dem menschlichen Körper entnommene Probe oder Flüssigkeit, insbesondere Blut, festgestellt wird, ob und wieviel mRNA (entsprechenden einem cDNA-Fragment nach einem der Ansprüche 1 bis 3) vorhanden ist. Dies erlaubt vor allem Diagnosen bei Erkrankungen, bei denen die zellvermittelte Immunantwort, vorzugsweise das Interferon-gamma-Signal eine Rolle spielt (z. B. Infektionen mit Viren, Parasiten, Pilzen und Bakterien, Tumorerkrankungen, Autoimmunerkrankungen, Transplantatabstoßung). Anstelle oder zusätzlich zur mRNA kann auch das entsprechende Protein bzw. entsprechende Antikörper dazu zur Diagnose herangezogen werden.

Darüber hinaus ermöglichen die erfindungsgemäßen cDNAs, mRNAs bzw. Proteine die Herstellung von Arzneimitteln, insbesondere gegen Erkrankungen bei denen das Interferon-gamma-Signal eine Rolle spielt. Diese Arzneimittel können folgende Elemente enthalten:
◆ Ein Vehikel und ein cDNA-Fragment nach einem der Ansprüche 1 bis 3 bzw. eine entsprechende mRNA.
◆ Ein Antisense-Oligonukleotid zur mRNA, die einem cDNA-Fragment nach einem der Ansprüche 1 bis 3 entspricht.
◆ Ein Protein nach Anspruch 9 oder 10.
◆ Einen die Wirkung des Proteins nach Anspruch 9 oder 10 hemmenden Stoff (Antikörper).

### Isolierung des Mig-beta Klons aus humanen Monocytomazellen (THP-1):

THP-1 Zellen (American type culture collection (ATCC), Rockville, Maryland, U.S.A.) wurden in AIM-V Serum-freiem Kulturmedium (Gibco BRL, Life Technologies, Wien, Osterreich) das 0.1 % Rinderserumalbumin (BSA, Fraktion V, Serva, Heidelberg, Deutschland), 2 mM L-Glutamin (Serva), 100 µg/ml Streptomycin und 100 U/ml Penicillin (Biological Industries, Kibbutz Beit Haemek, Israel) enthielt, für 7 Stunden mit 250 U/ml humanem rekombinantem Interferon-gamma stimuliert. Danach wurden die Zellen durch Zentrifugation und Waschen in Phosphat-gepufferter Salzlösung (PBS) isoliert, RNA mit Standardtechniken gewonnen (s. CHOMEZYNSKY, P., N. SACCHI: Single-step method of RNA isolation by acid guanidinium thiocyanate phenol chloroform ectraction. Annals of Biological Chemistry 162 (1987) 156-159.), und aus dieser mit Hilfe des Oligotex mRNA Kits (Quiagen, Hilden, Deutschland) Poly-A + mRNA hergestellt. Mit Hilfe von reverser Transkriptase und einem Oligo (dT) Primer wurde die mRNA in cDNA umgeschrieben, in den Lambda ZAP II Vektor (Stratagene, La Jolla, CA, U.S.A), der mit EcoRI geschnitten und dephosphoryliert worden war, ligiert, und mit dem Gigapack II Pack-Extrakt (Stratagene) in Lambda-Phagen verpackt. Die so hergestellte cDNA Library wurde mit einer mittels Polymerase-Kettenreaktion (PCR) hergestellten 550 bp Sonde gegen das durch Interferon-gamma induzierte Protein GTP-cyclohydrolase I durchsucht und positive Klone isoliert. Die Mig-beta cDNA wurde als eine neue cDNA-Sequenz gefunden, die mit dem für die Herstellung der cDNA Library verwendeten 12-Basenpaar EcoRI-Linker an die cDNA für GTP-cyclohydrolase I ligiert war. Aus diesem fusionierten Klon wurde durch Umklonieren die Mig-beta cDNA gewonnen.

### Detektion der Expression der Mig-beta mRNA mittels Northern Blot:

Total-RNA wird aus dem zu testenden Gewebe bzw. aus den zu testenden Zellen mittels Standardmethoden (s. CHOMEZYNSKY, P., N. SACCHI: Single-step method of RNA isolation by acid guanidinium thiocyante phenol chloroform extraction. Annals of Biological Chemistry 162 (1987) 156-159.) isoliert. Die RNA wird durch Elektrophorese in einem 1 % (w/v) Agarose, 6 % (v/v) Formaldehyd Gel getrennt, auf eine Nylon-membrane (Duralon-UV, Stratagene) übertragen und mittels UV-Licht an die Membran gebunden. Eine Sonde wird durch Restriktionsverdau (z. B. Xbal) aus dem die Mig-beta cDNA enthaltenden Plasmid gewonnen, nach Trennung in einem Agarosegel aus diesem isoliert (z. B. mit Hilfe eines Gel-Extraktionskits, Quiagen) und mit ³²P-dCTP und der Kleenow-Reaktion markiert. Die Nylonmembran mit der gebundenen RNA wird nun bei 65°C mit 10⁶ cpm/ml für 10-20 Stunden unter Verwendung von Standardprotokollen (s. Short Protocols in Molecular Biology, Ausubel, F. M., Brent, R., Kingston R. E., Moore, D. D., Seidman, J. G., Smith, J. A., Struhl, K., eds., John Wiley & Sons, New York, U.S.A., 1992) hybridisiert und gewaschen. BioMAX MS-1 Filme (Kodak) werden mit den Membranen unter Verwendung von Intensivierungsschirmen für 1 bis 6 Tage exponiert und dann entwickelt. Das Ausmaß der Schwärzung der Filme in einem Größenbereich entsprechend ca. 1500 Basenpaaren ist ein Maß für die Expression der Mig-beta mRNA.

### Herstellung des Mig-beta Chemokin-Precursors bzw. des Mig-beta Chemokins in Escherichia coli mit Hilfe des pet16b-Vektors und des His-Tag Fusionsproteinansatzes:

Zur Herstellung des Mig-beta Proteins, bzw. des Mig-beta-Precursor (mit Signalsequenz) Proteins in *Escherichia coli (E.coli)* unter Verwendung des pet16b-Vektors (Novagen, R & D Systems, Abingdon, U. K.) und des His-Tag Reinigungsansatzes wird wie folgt vorgegangen: Mit Hilfe der Polymerase-Kettenreaktion (PCR) wird mit den folgenden Primern eine für das Klonieren in den Vektor unter Verwendung der Ndel/Xhol Restriktionsenzyme geeignete Sequenz des Leserahmens erzeugt: Als Sense-Primer für den Mig-beta-Precursor (Seq. ID NO:1, Lagen 46 bis 327) dient die Sequenz GCAAAAAAACACATATGAGTGTGAAGGG, für Mig-beta (Seq. ID NO: 1, Lagen 109 bis 327) die Sequenz CAAGGCTTCCATATGTTCAAAAGAGG. Als Antisense-Primer dient sowohl für Mig-beta, als auch für den Mig-beta-Precursor die Sequenz CATATGCTCTTCTCGAGGACTTTATATGTT. Die PCR-Produkte werden mit Hilfe des TA-Cloning Kits (Clontech, Palo-Alto, CA, U.S.A.) kloniert, mit NdelXhol verdaut, mit Hilfe eines Agarosegels gereinigt, aus diesem Gel gewonnen und in den mit Ndol/Xhol verdauten, desphosphorylierten pet16b-Vektor ligiert. Unter Verwendung eines Protease-negative *E. coli* Stammes (BL21DE3) wird das Protein exprimiert, mit Hilfe des fusionierten His-Tags gereinigt und dieser mit Faktor Xa abgespalten. Dazu werden die das Plasmid tragenden *E. coli* Bakterien mit 1-2 mM Isopropyl-1-thio-β-D-galaktosid (IPTG) für 2-4 Stunden inkubiert, die Zellen lysiert und das mit dem His-Tag versehene Protein mit Hilfe des Ni-NTA Harzes (Quiagen) mit Hilfe von Standardprotokollen (The QIAexpressionist, Quiagen) isoliert. Der an das Mig-beta bzw. an den Mig-beta-Precursor fusionierte His-Tag wird schließlich mit Faktor Xa abgespalten.

Das beiliegende Sequenzprotokoll und dessen Angaben sind integrierender Bestandteil der vorliegenden Beschreibung.

### Referenzen:

1. Bleul, C.C., Farzan, M., Choe, H., Parolin, C., Clark-Lewis, I., Sodroski, J., & Springer, T.A. (1996). The lymphocyte chemoattractant SDF-1 is a ligand for LESTR/fusin and blocks HIV-1 entry. Nature 382,829-833.
2. Oberlin, E., Amara, A., Bachelerie, F., Bessia, C., Virelizier, J.L., Arenza Seisdedos, F., Schwartz, O., Heard, J.M., Clark-Lewis, I., Legler, D.F., Loetscher, M., Baggiolini, M., & Moser, B. (1996). The CXC chemokine SDF-1 is the ligand for LESTR/fusin and prevents infection by T-cell-line adapted HIV-1. Nature 382, 833-835.
3. Farber, J. M. (1997) Mig and IP-10: CXC chemokines that target lymphocytes. J. Leukocyte Bio. 61:246-257.

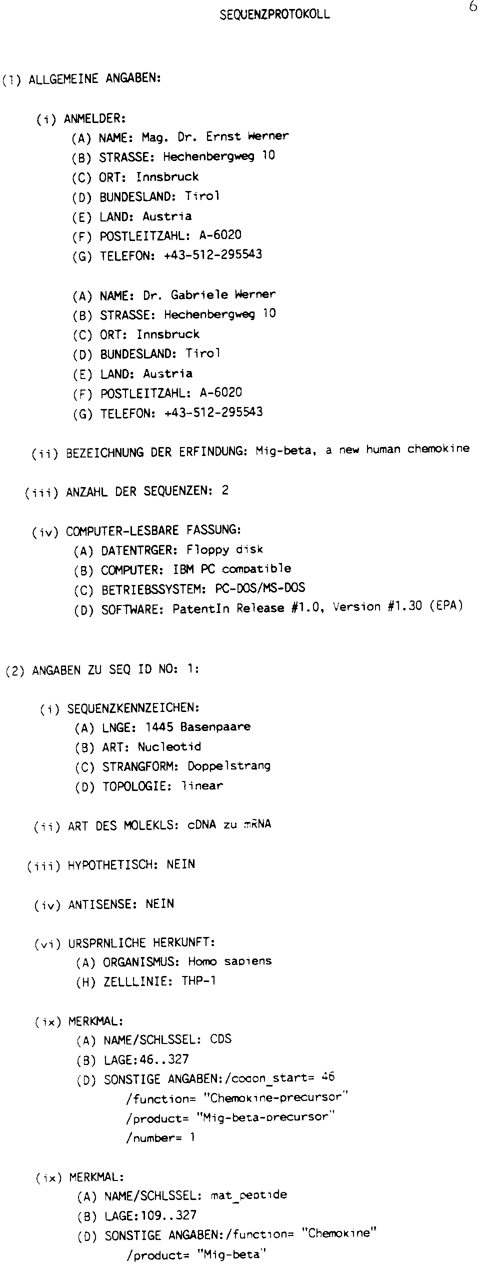

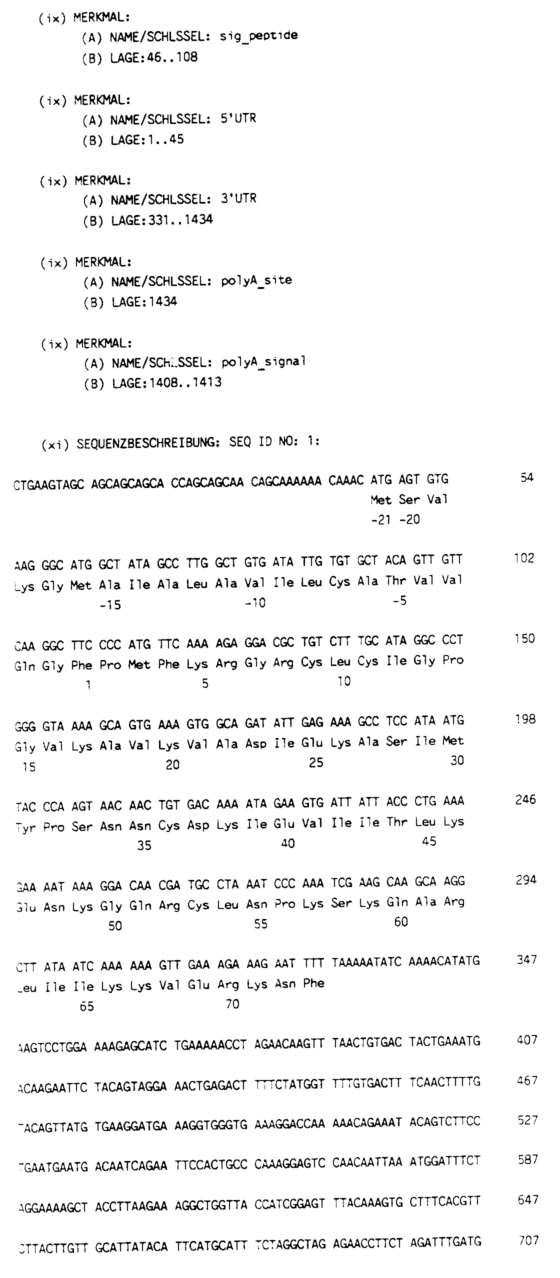

CTTACAACTA TTCTGTTGTG ACTATGAGAA CATTTCTGTC TCTAGAAGTC ATCTGTCTGT 767

ATTGATCTTT ATGCTATATT ACTATCTGTG GTTACGGTGG AGACATTGAC ATTATTACTG 827

GAGTCAAGCC CTTATAAGTC AAAAGCATCT ATGTGTCGTA AAACATTCCT CAAACATTTT 887

TTCATGCAAA TACACACTTC TTTCCCCAAA CATCATGTAG CACATCAATA TGTAGGGAGA 947

CATTCTTATG CATCATTTGG TTTGTTTTAT AACCAATTCA TTAAATGTAA TTCATAAAAT 1007

GTACTATGAA AAAAATTATA CGCTATGGGA TACTGGCAAA AGTGCACATA TTTCATAACC 1067

AAATTAGTAG CACCAGTCTT AATTTGATGT TTTTCAACTT TTATTCATTG AGATGTTTTG 1127

AAGCAATTAG GATATGTGTG TTTACTGTAC TTTTTGTTTT GATCCGTTTG TATAAATGAT 1187

AGCAATATCT TGGACACATC TGAAATACAA AATGTTTTTG TCTACCAAAG AAAAATGTTG 1247

AAAAATAAGC AAATGTATAC CTAGCAATCA CTTTTACTTT TTGTAATTCT GTCTCTTAGA 1307

AAAATACATA ATCTAATCAA TTTCTTTGTT CATGCCTATA TACTGTAAAA TTTAGGTATA 1367

CTCAAGACTA GTTTAAAGAA TCAAAGTCAT TTTTTTCTCT AATAAACTAC CACAACCTTT 1427

CTTTTTTAAA AAAAAAAA 1445

(2) ANGABEN ZU SEQ ID NO: 2:

(i) SEQUENZKENNZEICHEN:
(A) LNGE: 94 Aminosuren
(B) ART: Aminosure
(D) TOPOLOGIE: linear

(ii) ART DES MOLEKLS: Protein
(xi) SEQUENZBESCHREIBUNG: SEQ ID NO: 2:

Met Ser Val Lys Gly Met Ala Ile Ala Leu Ala Val Ile Leu Cys Ala
-21 -20 -15 -10

Thr Val Val Gln Gly Phe Pro Met Phe Lys Arg Gly Arg Cys Leu Cys
-5 1 5 10

Ile Gly Pro Gly Val Lys Ala Val Lys Val Ala Asp Ile Glu Lys Ala
15 20 25

Ser Ile Met Tyr Pro Ser Asn Asn Cys Asp Lys Ile Glu Val Ile Ile
30 35 40

Thr Leu Lys Glu Asn Lys Gly Gln Arg Cys Leu Asn Pro Lys Ser Lys
45 50 55

Gln Ala Arg Leu Ile Ile Lys Lys Val Glu Arg Lys Asn Phe
60 65 70

## Patentansprüche

1. Ein cDNA-Fragment wie es im Sequenzprotokoll mit SEQ ID NO:1 definiert ist.

2. Ein cDNA-Fragment wie es im Sequenzprotokoll mit SEQ ID NO:1 in den Lagen 46 bis 327 definiert ist.

3. Ein cDNA-Fragment wie es im Sequenzprotokoll mit SEQ ID NO:1 in den Lagen 109 bis 327 definiert ist.

4. DNA-Transfervektor, dadurch gekennzeichnet, daß er eine cDNA-Desoxynukleotidsequenz enthält, die einem Gen oder einem DNA-Fragment entspricht, wie es in einem der Ansprüche 1 bis 3 definiert ist.

5. DNA-Fragment oder DNA-Transfervektor nach einem der Ansprüche 1 bis 4, dadurch gekennzeichnet, daß eines oder mehrere natürliche Codons oder ihre cDNA-Äquivalente durch einen anderen Codon ersetzt sind, der für dieselbe Aminosäure kodiert.

6. DNA-Transfervektor nach Anspruch 4 oder 5, dadurch gekennzeichnet, daß er ein bakterielles Plasmid ist.

7. DNA-Transfervektor nach Anspruch 4 oder 5, dadurch gekennzeichnet, daß er eine Bakteriophagen-DNA ist.

8. Zelle, transformiert von einem Transfervektor nach einem der Ansprüche 4 bis 7.

9. Protein mit einer 94 Aminosäuren umfassenden Aminosäuresequenz wie sie im Sequenzprotokoll mit SEQ ID NO:2 definiert ist.

10. Protein mit einer 73 Aminosäuren umfassenden Aminosäuresequenz wie sie im Sequenzprotokoll mit SEQ ID NO:2 in den Lagen 1 bis 73 definiert ist.

11. Diagnoseverfahren bei dem in einer dem menschlichen Körper entnommenen Probe oder Flüssigkeit, insbesondere Blut, festgestellt wird, ob und wieviel mRNA, die einem cDNA-Fragment nach einem der Ansprüche 1 bis 3 entspricht, vorhanden sind.

12. Diagnoseverfahren bei dem in einer dem menschlichen Körper entnommenen Probe oder Flüssigkeit, insbesondere Blut, festgestellt wird, ob und wieviel vom Protein gemäß Anspruch 9 oder 10 oder einem Antikörper dazu vorhanden ist.

13. Arzneimittel zur therapeutischen Behandlung, dadurch gekennzeichnet, daß es ein Vehikel und ein cDNA-Fragment nach einem der Ansprüche 1 bis 3 eine entsprechende mRNA enthält.

14. Arzneimittel zur therapeutischen Behandlung, dadurch gekennzeichnet, daß es ein Antisense-Oligonukleotid zur mRNA enthält, die einem cDNA-Fragment nach einem der Ansprüche 1 bis 3 entspricht.

15. Arzneimittel zur therapeutischen Behandlung, dadurch gekennzeichnet, daß es ein Protein nach Anspruch 9 oder 10 enthält.

16. Arzneimittel zur therapeutischen Behandlung, dadurch gekennzeichnet, daß es einen die Wirkung des Proteins nach Anspruch 9 oder 10 hemmenden Stoffes enthält.

17. Verwendung eines cDNA-Fragments nach einem der Ansprüche 1 bis 3, einer entsprechenden mRNA, eines zugehörigen Antisense-Oligonukleotids, eines Proteins nach Anspruch 9 oder 10 oder eines die Wirkung dieses Proteins hemmenden Stoffes zur Herstellung eines Arzneimittels gegen Erkrankungen bei denen die zellvermittelte Immunantwort, vorzugsweise das Interferon-gamma-Signal eine Rolle spielt.

18. Verwendung eines cDNA-Fragments nach einem der Ansprüche 1 bis 3, einer entsprechenden mRNA, eines zugehörigen Antisense-Oligonukleotids, eines Proteins nach Anspruch 9 oder 10 oder eines die Wirkung dieses Proteins hemmenden Stoffes zur Herstellung eines Arzneimittels gegen Infektionen mit Viren, Parasiten, Pilzen oder Bakterien, oder gegen Tumorerkrankungen, Autoimmunerkrankungen bzw. Tranplantatabstoßung.
